**Europäisches Patentamt**

**European Patent Office**

**Office européen des brevets**

(11) Numéro de publication: **0 271 369 B1**

# FASCICULE DE BREVET EUROPEEN

(45) Date de publication de fascicule du brevet:
03.07.91

(51) Int. Cl.⁵: **G01R 33/48**, A61B 6/00

(21) Numéro de dépôt: 87402256.9

(22) Date de dépôt: 09.10.87

(54) **Dispositif optique de détection simultanée des mouvements du coeur et de la respiration.**

(30) Priorité: **14.10.86 FR 8614260**

(43) Date de publication de la demande:
**15.06.88 Bulletin 88/24**

(45) Mention de la délivrance du brevet:
**03.07.91 Bulletin 91/27**

(84) Etats contractants désignés:
**BE DE GB IT NL SE**

(56) Documents cités:
**EP-A- 0 072 601**
**FR-A- 2 473 872**
**US-A- 4 210 029**
**US-A- 4 461 368**

**AMERICAN JOURNAL OF ROENTGENOLOGY,
vol. 143, déecembre 1984, pages 1175-1182,
Baltimore, US; R.L. EHMAN et al.: "Magnetic
resonance imaging with respiratory gating:
techniques & advantages"**

(73) Titulaire: **THOMSON-CSF**
**51, Esplanade du Général de Gaulle**
**F-92800 Puteaux(FR)**

(72) Inventeur: **Kretschmer, Sylvian**
**THOMSON-CSF SCPI 19, avenue de Messine**
**F-75008 Paris(FR)**
Inventeur: **Do-Huu, Jean-Paul**
**THOMSON-CSF SCPI 19, avenue de Messine**
**F-75008 Paris(FR)**
Inventeur: **Micheron, François**
**THOMSON-CSF SCPI 19, avenue de Messine**
**F-75008 Paris(FR)**

(74) Mandataire: **Schmit, Christian Norbert Marie
et al**
**Cabinet Ballot-Schmit 7, rue Le Sueur**
**F-75116 Paris(FR)**

Rank Xerox (UK) Business Services

Description

La présente invention concerne un dispositif optique de détection simultanée des mouvements du coeur et de la respiration et son utilisation à la synchronisation d'appareils d'acquisition d'images à résonance magnétique nucléaire plus communément désignés par IRM.

Il est connu, pour améliorer la qualité des images fournies par ces appareils de les synchroniser sur les rythmes cardiaques et respiratoires des patients examinés, à l'aide de signaux provenant d'électrocardiographes.

Cependant les électrodes et câbles de liaison métalliques qui servent au transport des signaux de synchronisation déforment les lignes de champ des puissants champs magnétiques et radioélectrique rayonnés et la qualité des images obtenues est affectée. Il en résulte un inconvénient majeur pour les médecins qui risquent d'établir de faux diagnostics.

Selon une variante de réalisation connue du procédé précité, la synchronisation est effectuée à partir de signaux provenant de stétoscopes acoustiques et pneumatiques. Ceux-ci ont l'avantage sur les électrocardiographes d'être non interférants avec les champs magnétiques et électromagnétiques. Mais ils restent sensibles aux bruits acoustiques ambiants et le temps de propagation de l'onde de pression dans les tubes de liaison qui les relient aux appareils IRM, entraine un déphasage rédhibitoire et difficile à compenser entre des signaux de synchronisation et les activités cardiaques et respiratoires correspondantes.

La demande de certificat d'utilité français 2 473 872 décrit dans un même but un système de détection de type électrooptique. Cependant ce système est peu sensible aux faibles mouvements ou, alors, trop sensible aux forts mouvements. D'autres systèmes, notamment ceux décrits dans l'article de R. L. EHMMAN et AI : "Magnetic resonance imaging with respiratory gating techniques et advantages", American Journal of Roengenology, Vol 143 Décembre 1984, pages 1175 - 1182, ne sont pas non plus capables, avec un seul appareil de proposer une sensibilité comparable pour des mouvements d'amplitudes très différents.

Le but de l'invention est de pallier les inconvénients précités.

A cet effet, l'invention a pour objet, un dispositif optique pour la détection simultanée des mouvements du coeur et de la respiration caractérisé en ce qu'il comprend un capteur comportant un miroir mobile couplé à des moyens pour déplacer le miroir par les mouvements du coeur et du thorax du patient, un générateur de lumière pour éclairer le miroir ainsi que des moyens électrooptiques pour convertir l'intensité des rayons lumineux réfléchis par le miroir en un signal électrique.

L'invention a également pour objet une utilisation du dispositif précité à la synchronisation des appareils d'acquisition d'images à résonance magnétique nucléaire.

D'autres caractéristiques et avantages de l'invention apparaîtront ci-après à l'aide de la description faite en regard des dessins annexés qui représentent :

- la figure 1 le principe de réalisation du dispositif selon l'invention ;
- la figure 2 un graphe illustrant le fonctionnement du dispositif de la figure 1 ;
- la figure 3 un mode de réalisation complet du capteur ;
- la figure 4 un mode de réalisation de moyens électroniques de traitement.
- la figure 5 un mode de réalisation d'une logique de mise en forme des signaux de synchronisation ;
- les figures 6 et 7 des diagrammes de temps pour illustrer le fonctionnement de la logique représentée à la figure 5.

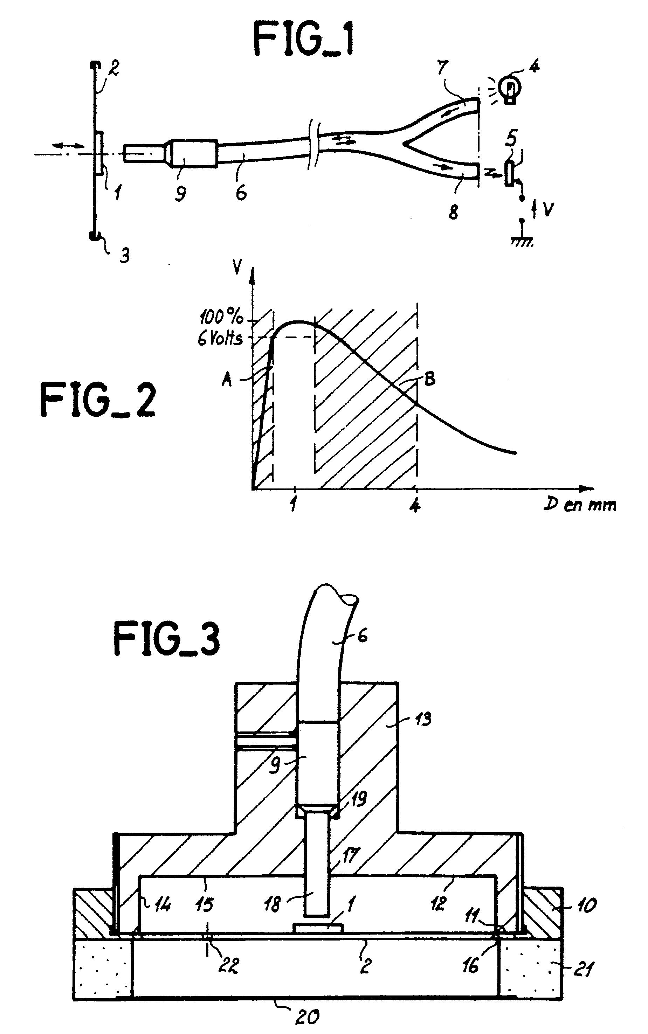

Le dispositif selon l'invention qui est représenté de façon simplifiée suivant le schéma de principe de la figure 1, permet de recueillir les bruits cardiaques comme le ferait un stétoscope acoustique en phonocardiographie stétoscopique avec cependant l'avantage qu'il supprime les retards de propagation inhérents aux ondes acoustiques se propageant dans le tube de liaison de ces stétoscopes.

Suivant le mode de réalisation représenté, le capteur du dispositif selon l'invention est constitué par un miroir 1 collé sur une membrane 2 qui est maintenue tendue par ses bords à un cadre 3. Le miroir 1 est éclairé par un générateur de lumière 4, constitué éventuellement par une diode laser ou tout dispositif équivalent, et la lumière, réfléchie par le miroir, est renvoyée en direction d'un détecteur électrooptique 5, constitué éventuellement par un phototransistor ou tout dispositif équivalent. La lumière est transportée dans les deux sens de propagation par un guide de lumière 6, en forme de Y, interposé entre, d'une part, le miroir 1 et d'autre part, le générateur de lumière 4 et le détecteur électrooptique .5. Pour effectuer cette propagation, le guide de lumière 6 est formé par deux faisceaux de fibres optiques distincts, 7 et 8, liés ensemble à une de leurs extrémités, en regard du miroir 1 par un fourreau 9. Les autres extrémités des faisceaux 7 et 8 sont séparées l'une de l'autre, pour permettre l'introduction de la lumière fournie par le générateur 4 dans l'un des faisceaux, (le faisceau 7 par exemple), et sa détection à l'extrémité de l'autre faisceau (le faisceau 8 par exemple), par le détecteur électrooptique 5. A titre indicatif mais non limitatif, le guide de lumière 6 pourra éventuellement être constitué par une sonde à fibres optiques du type commercialisé sous la référence BFS-CGP2 par la Société de droit français FORT.

La lumière réfléchie par le miroir 1 et parvenant à l'extrémité du faisceau 8 provoque l'apparition à la sortie du détecteur électrooptique 5 d'une tension V dont l'amplitude est modulée en fonction du déplacement D du miroir 1 suivant la direction longitudinale du guide de lumière 6. Une courbe représentant l'évolution de la tension V en fonction du déplacement D est représentée à la figure 2. Cette courbe se caractérise par deux zones à peu près linéaires A et B, présentant deux sensibilités différentes.

Dans la zone A, la tension V évolue entre une valeur nulle et une valeur d'environ 6 volts proche de sa valeur maximale pour des déplacements D compris entre 0 et 0,5 mm, et la sensibilité est d'environ 12 volts par millimètre. Par contre, dans la zone B, la tension évolue dans le sens contraire depuis une valeur 6 volts proche de la valeur maximale jusqu'à une valeur atténuée d'environ de moitié pour des déplacements compris entre 1 et 4 mm et la sensibilité est d'environ 1 volt par millimètre. Ces résultats montrent que le dispositif de l'invention peut être avantageusement utilisé à la détection du mouvement respiratoire chez l'homme, car les déplacements du miroir 1 qui caractérisent la zone B, ont des amplitudes tout à fait comparables à celles que l'on peut escompter d'un mouvement respiratoire en plaçant directement la membrane 2 sur la poitrine d'un patient. Mais, il apparait aussi que la sensibilité de cette zone est trop faible pour mettre en évidence les pulsations cardiaques qui provoquent à la surface du thorax des déplacements de quelques centièmes de millimètre seulement. Dans ces conditions on ne peut obtenir, en exploitant la zone B, qu'un signal utile de quelques dizaines de millivolt qui serait très difficile à séparer du bruit électronique ambiant. La zone A par contre offre cette possibilité, la sensibilité dans cette zone étant suffisante pour extraire un signal utile supérieur au bruit propre des circuits électroniques. Cependant la très faible dynamique des déplacements du miroir, inférieure à 0,5 mm, interdit la mise en contact direct de la membrane 2 sur la poitrine des patients. Cette difficulté est surmontée selon l'invention en intercalant entre la membrane 2 et la poitrine du patient une couronne intermédiaire. Un exemple de capteur réalisé suivant ce principe est représenté à la figure 3 où les éléments homologues à ceux de la figure 1 sont repérés avec les mêmes références. Suivant cet exemple, la membrane 2 est fixée, à l'aide d'une bague filetée 10, sur le bord extérieur 11 d'une cavité cylindrique 12 réalisée dans le corps d'une pièce support 13. La cavité 12 est délimitée par une paroi cylindrique 14 et deux surfaces planes parallèles, perpendiculaires à l'axe de révolution de la cavité. Une surface plane est constituée par le fond 15 de la cavité et la deuxième surface plane est constituée par la membrane 2 maintenue en appui sur le bord extérieur 11 par un épaulement 16 de la bague 10.

Le fond 15 est percé en son centre et dans la direction perpendiculaire à son plan, d'un trou 17 dans lequel est engagé l'extrémité 18 du guide de lumière 6. Le trou 17 comporte également un épaulement 19 sur lequel est appuyée une extrémité du manchon 9. L'extrémité 18 du guide de lumière 6 débouche à l'intérieur de la cavité 12. La membrane 2 est positionnée sur les bords de la cavité pour que le miroir 1 qu'elle supporte soit directement en regard de l'extrémité 18 du guide de lumière. Pour permettre la détection des mouvements cardiaques sans que ceux-ci soient perturbés par le mouvement respiratoire une deuxième membrane 20 est couplée à la première membrane 2 par l'intermédiaire d'un cadre support ou d'une couronne souple 21 fixée en appui sur la bague 10 et un trou 22 de faible dimension est percé dans la membrane 2 pour équilibrer la pression de l'air de part et d'autre de la membrane 2, et donner à l'ensemble le comportement d'un filtre acoustique passe-haut afin d'éliminer les fréquences inférieures à celles de la respiration dues, par exemple, aux variations de température.

En cours d'utilisation, la membrane 20 est maintenue en contact avec la peau par une sangle, non représentée, qui est serrée autour du thorax en s'appuyant sur le corps de la pièce support 13. Ceci permet à l'onde acoustique cardiaque d'atteindre la membrane 2 à travers la membrane 20, et au mouvement respiratoire d'exercer une pression par l'intermédiaire de la sangle qui écrase plus ou moins la couronne souple 21 sur le thorax. Ces mouvements sont transmis au miroir 1 par le volume d'air emprisonné entre les deux membranes 20 et 2. Naturellement pour l'application IRM envisagée l'ensemble du capteur qui vient d'être décrit doit être réalisé en utilisant des matériaux non magnétiques et à ce titre on pourra utiliser des matières plastiques comme celle connue sous la marque de fabrique DELRIN, par exemple.

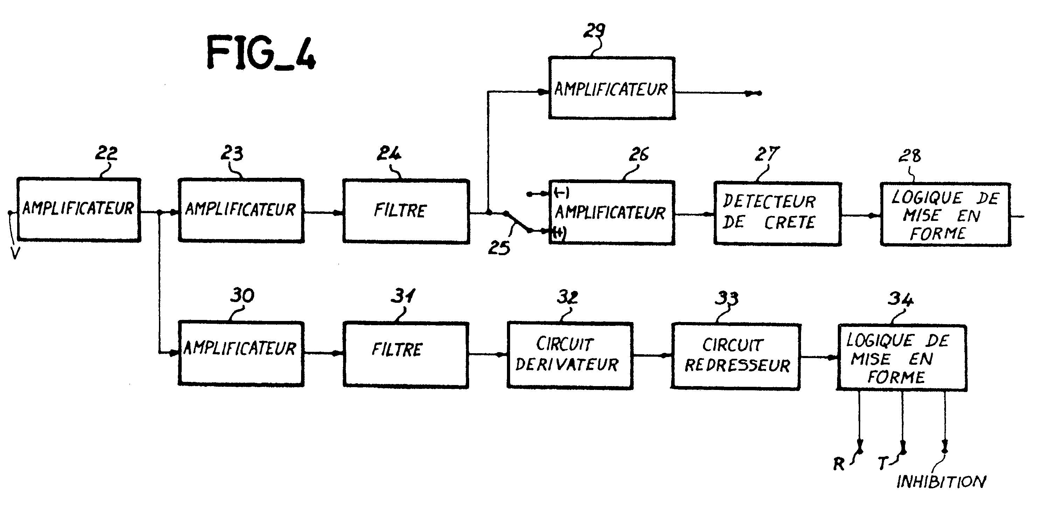

Un mode de réalisation de moyens électroniques de traitement permettant l'utilisation du dispositif selon l'invention à la synchronisation d'appareils d'acquisition d'images à résonance magnétique nucléaire est décrit ci-après à l'aide de la figure 4. Ces moyens comprennent, une première voie composée des éléments 23 à 29 dont le rôle est d'extraire les signaux représentatifs du rythme respiratoire et une deuxième voie composée des éléments 30 à 34 dont le rôle est d'extraire, les signaux représentatifs du rythme cardiaque, du signal V fourni par le capteur photoélectrique 5 de la figure 1 et amplifié par un amplificateur 22 commun aux deux voies.

La première voie comprend un amplificateur à gain réglable 23, un filtre passe-bas 24, un inverseur de polarité composé d'un interrupteur 25 et d'un amplificateur différentiel 26, un détecteur de crête 27, une logique de mise en forme 28 et un amplificateur 29.

L'amplificateur 23 est couplé à la sortie de l'amplificateur 22 et permet l'adaptation de la première voie de traitement à la sensibilité du capteur précédemment décrit. Le filtre passe-bas 24 a une fréquence de coupure de 4 hertz et permet d'atténuer les bruits cardiaques pour privilégier le signal provenant du thorax dû à la respiration et dont la fréquence est de l'ordre de 0,2 hertz. Il reçoit les signaux fournis par l'amplificateur 23 et les restitue filtrés sur les entrées inverseuses marquées "-" ou non inverseuses marquées "+" de l'amplificateur 26 au travers de l'interrupteur 25. L'action de l'interrupteur 25 sur les entrées marquées "-" ou "+" de l'amplificateur 26 permet de choisir le point de synchronisation soit à l'inspiration soit à l'expiration du mouvement respiratoire. La sortie de l'amplificateur 26 est couplée à l'entrée du détecteur de crête 27 pour obtenir de façon précise et reproductible l'instant marquant le début d'une inspiration ou d'une expiration du cycle respiratoire, les impulsions correspondantes sont éventuellement mises en forme par la logique de mise en forme 28. Enfin, un amplificateur-adaptateur d'impédance 29 est couplé également à la sortie du filtre 24 de façon à restituer le signal analogique correspondant au cycle respiratoire.

La deuxième voie constituée des éléments 30 à 34 comprend, reliés dans cet ordre en série, un amplificateur à réglage de gain 30, un filtre passe-bande 31 ayant par exemple des fréquences de coupures de 0,5 et 200 hertz, un circuit dérivateur 32 analogique d'ordre 2, un circuit redresseur 33 et une logique de mise en forme 34. Comme pour la première voie, l'amplificateur 30 est couplé à la sortie de l'amplificateur 22 et possède un gain réglable pour adapter la chaine de traitement de la deuxième voie à chaque sensibilité de capteur utilisé. Le filtre passe-bande 31 supprime du signal fourni par l'amplificateur 30, le signal respiratoire en basse fréquence, les parasites hautes fréquences et les bruits ambiants au-dessus de 200 hertz en laissant passer le signal utile représentatif du rythme cardiaque dont la fréquence est comprise dans la bande utile et n'excéde pas 150 hertz. Le circuit dérivateur analogique 32 reçoit des impulsions cardiaques filtrées par le filtre 31 pour fournir les deux impulsions correspondantes aux bruits B1 et B2 significatifs des ondes R et T définies en électrocardiographie comme étant les ondes de fin de diastole et de systole respectivement. Après redressement par le redresseur 33, la logique de mise en forme 34 assure la suppression des impulsions parasites ainsi que la sélection par deux voies différentes des signaux correspondant respectivement aux ondes R et T. Un autre dispositif logique situé à l'intérieur de la logique de mise en forme 34 permet de générer le signal d'inhibition.

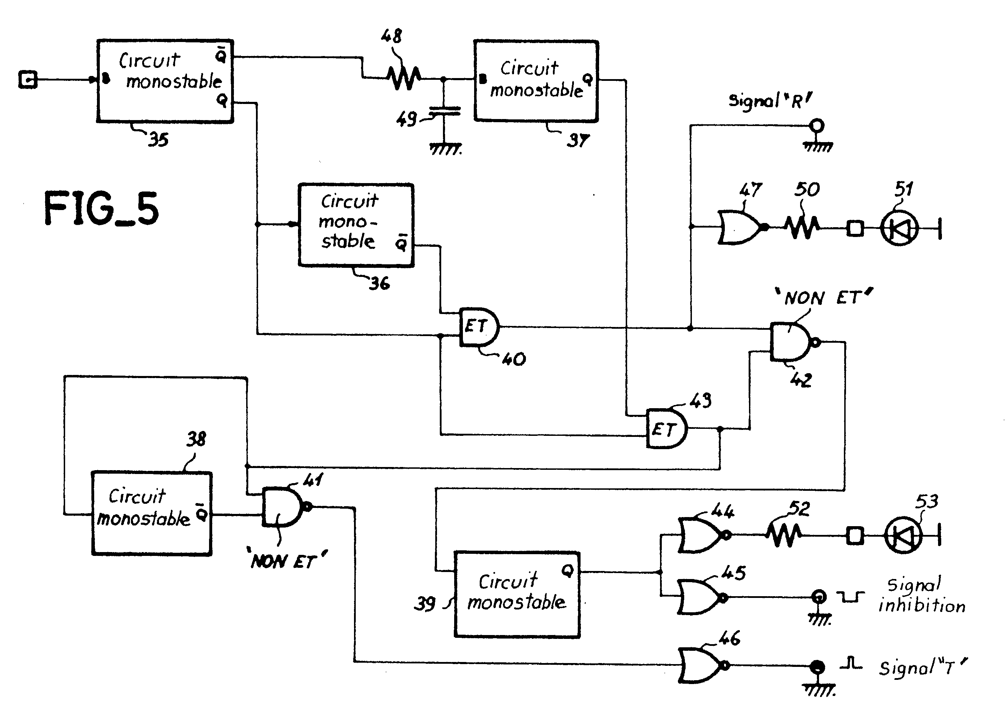

Un mode de réalisation de la logique de mise en forme 34 et son fonctionnement sont décrits ci-après à l'aide des figures 5, 6 et 7.

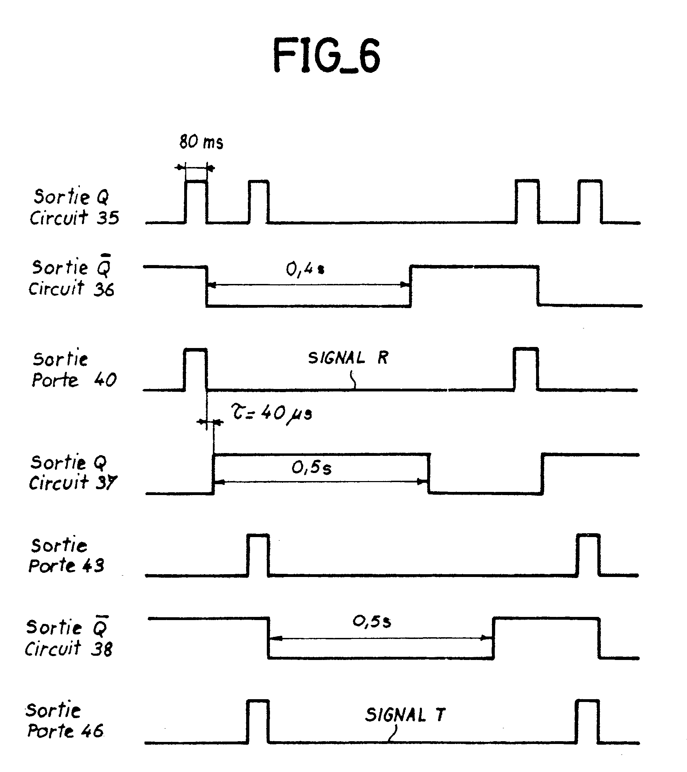

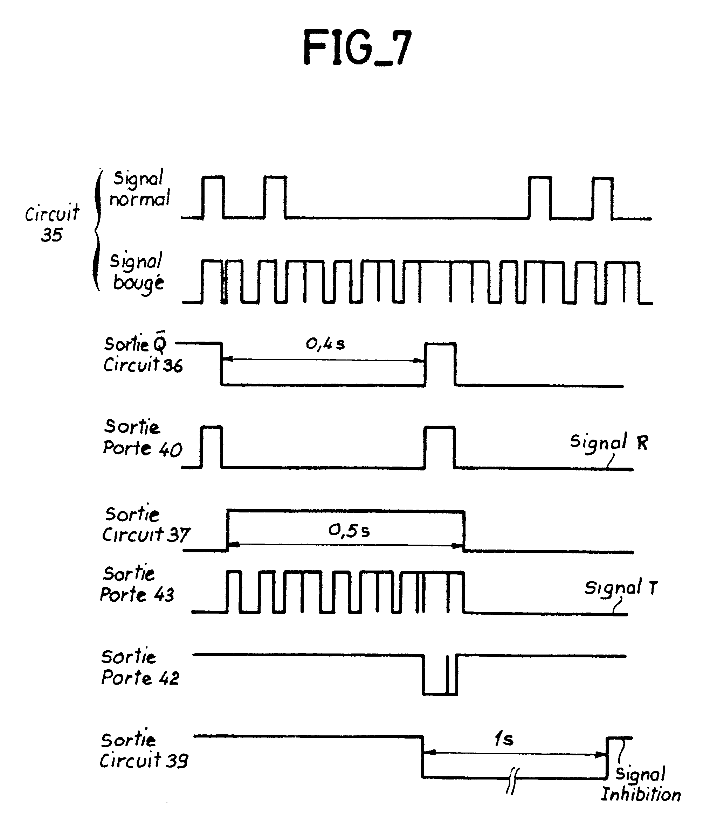

La logique de mise en forme 34 représentée à la figure 5 comprend un ensemble de circuits monostables 35 à 39 couplés à un ensemble de portes logiques 40 à 47. Une sortie directe marquée Q du circuit monostable 35 est reliée directement à l'entrée du circuit monostable 36 et à une première entrée de la porte 40 formée par une porte ET à deux entrées dont la deuxième entrée est reliée à la sortie complémentaire marquée $\overline{Q}$ du circuit monostable 36. L'entrée du circuit monostable 37 est reliée à la sortie complémentaire marquée $\overline{Q}$ du circuit monostable 35 au travers d'un circuit à retard formé par une résistance 48 et un condensateur 49. Les portes 41 et 42 sont des portes "non ET" à deux entrées. La porte 43 est une porte ET, elle possède deux entrées qui sont reliées respectivement à une sortie marquée Q du circuit monostable 37 et à la sortie marquée Q du circuit monostable 35. La porte ET 40 élabore le signal R et applique ce signal sur une première entrée de la porte "non ET" 42 et sur un dispositif de visualisation constitué par la porte 47 une résistance 50 et une diode électroluminescente 51 montées en série. La deuxième entrée de la porte "non ET" 42 est reliée à la sortie de la porte ET 43, à une entrée du circuit monostable 38 et à une première entrée de la porte "non ET" à deux entrées 41. La sortie complémentaire, marquée $\overline{Q}$ du circuit monostable 38 est reliée à une deuxième entrée de la porte "non ET" 41 dont la sortie fournie le signal T au travers de la porte inverseuse 46. Le circuit monostable 39 est déclenchée sur son entrée par le signal fourni par la sortie de la porte "non ET" 42, et sa sortie marquée Q fournit le signal d'inhibition, d'une part, à un dispositif de visualisation formé par la porte 44 une résistance 52 et une diode électroluminescente montées en série et d'autre part, à l'entrée de la porte 45. Les circuits monostables ont des durées de temporisation respectivement de 80 ms ; 0,4 s ; 0,5 s ; 0,46 s ; et 1 s. Le circuit de retard constitué par les résistances 48 et le condensateur 49 à une structure de filtre passe bas du premier ordre et possède une constante de temps de 40 μs. Les diagrammes des temps représentant l'élaboration des signaux R et T d'une part et d'inhibition d'autre part, sont représentés respectivement aux figures 6 et 7. Sur ces figures et notamment sur la figure 7 on peut voir que la sortie du circuit 35 présente un signal comprenant les impulsions R et T du rythme cardiaque avec éventuellement quelques impulsions indésirables reflètant un bougé du patient. Le circuit 36 associé à la porte 40 permet d'extraire uniquement l'impulsion R. Les circuits 37 et 38 associés à la porte 43 permettent d'extraire l'impulsion T.

La formation du signal d'inhibition est assurée par la porte 42 et le circuit 39. Elle met à profit le fait qu'en cas de "bougé" du patient, le signal de dérivée fourni par le circuit différentiateur 32 se présente sous la forme d'un train d'impulsions avec les impulsions R et T présentes simultanément. La porte 42 déclenche alors un signal d'inhibition dont la durée est fixée à une seconde par le circuit 39.

## Revendications

1. Dispositif optique pour la détection simultanée des mouvements du coeur et de la respiration caractérisé en ce qu'il comprend d'une part un capteur comportant un miroir mobile (1) couplé à des moyens (2, 3, 20, 21) pour déplacer le miroir par les mouvements du coeur et du thorax du patient, et d'autre part, un générateur de lumière couplé à un guide de lumière pour éclairer le miroir (1), des moyens électrooptiques (5) aussi couplés à ce guide de lumière pour convertir l'intensité des rayons lumineux réfléchis par le miroir (1) en un signal électrique, ainsi que des moyens de traitement pour convertir ce signal électrique en des signaux représentatifs des mouvements du coeur et de la respiration.

2. Dispositif selon la revendication 1, caractérisé en ce que les moyens pour déplacer le miroir sont constitués par une première membrane (2) supportant le miroir (1) qui est maintenue écartée du thorax par un cadre support (21).

3. Dispositif selon la revendication 2, caractérisé en ce qu'il comprend une deuxième membrane (20) fixée sur le cadre support parallèlement à la première membrane (2).

4. Dispositif selon les revendications 2 et 3 caractérisé en ce que le cadre support (21) est réalisé dans un matériau déformable.

5. Dispositif selon l'une quelconque des revendications 1 à 4, caractérisé en ce que la première membrane (2) obture une cavité (12) et est percée d'un trou (22) pour faire communiquer l'espace compris entre les deux membranes (2,20) avec la cavité (12) et réaliser avec la cavité (12) et la deuxième membrane (20) un filtre acoustique passe-haut.

6. Dispositif selon l'une quelconque des revendications 1 à 5, caractérisé en ce que le miroir (1) est éclairé par le générateur de lumière au moyen d'une fibre optique (7).

7. Dispositif selon l'une quelconque des revendications 1 à 6, caractérisé en ce que les rayons lumineux réfléchis par le miroir (1) sont transmis aux moyens électrooptiques (5) à conversion au moyen d'une fibre optique (8).

8. Dispositif selon l'une quelconque des revendications 1 à 7, caractérisé en ce que le capteur est maintenu par une sangle légèrement serrée autour du thorax.

9. Dispositif selon l'une quelconque des revendications 5 à 8 adapté à la synchronisation d'un appareil d'acquisition d'images à résonance magnétique nucléaire, caractérisé en ce qu'il comporte des moyens pour relier l'appareil d'acquisition d'images au dispositif optique pour la détection des mouvements du coeur, ces moyens pour relier étant reliés à des moyens électroniques de traitement (22,..., 34) fournissant, à partir du signal électrique fourni par les moyens électrooptiques (5), des signaux représentatifs des rythmes cardiaques, respiratoires et du "bougé" du patient.

10. Dispositif selon la revendication 9 caractérisé en ce que les moyens électroniques de traitement comprennent une première voie de traitement (23,...,29) pour extraire les signaux représentatifs du rythme respiratoire et une deuxième voie de traitement (30,...,34) pour extraire les signaux représentatifs du rythme cardiaque.

11. Dispositif selon la revendication 10, caractérisé en ce que la première voie comprend reliés dans cet ordre, en série, un amplificateur (23) avec réglage de gain permettant l'adaptation à la sensibilité du capteur, un filtre passe-bas (24) atténuant les bruits cardiaques pour privilégier le signal provenant du thorax dû à la respiration, un inverseur de polarité (25) pour choisir l'instant de synchronisation à l'inspiration ou à l'expiration et un détecteur de tension crête (27) pour obtenir un instant précis et reproductible du cycle respiratoire.

12. Dispositif selon les revendications 10 et 11, caractérisé en ce que la deuxième voie comprend reliés dans cet ordre en série un amplificateur à réglage de gain (30), un filtre passe-bande bande (31) pour supprimer le signal respiratoire et les signaux parasites et/ou les bruits ambiants au-dessus des fréquences de battement les plus hautes du coeur, un circuit dérivateur analogique (32) d'ordre 2 fournissant deux impulsions correspondantes aux bruits B1 et B2 significatifs des ondes R et T connues de l'électrocardiographie et une logique de mise en forme (34).

13. Dispositif selon la revendication 12, caractérisé en ce qu'il comporte une logique de mise en forme (34) pour fournir un signal d'inhibition en cas de bougé du patient.

## Claims

1. An optical device for the simultaneous detection of cardiac and respiratory movement, characterized in that it on the one hand comprises a sensor with a movable mirror (1) coupled with means (2, 3, 20 and 21) in order to displace the mirror by dint of the cardiac and thoracic movement of the patient, and on the other hand a light generator coupled with a light guide in order to shine on the mirror (1), electrooptic means (5) also coupled with this light guide in order to convert the intensity of the light rays reflected by the mirror (1) into an electrical signal, and processing means in order to convert this electrical signal into signals representing the cardiac and respiratory movement.

2. The device as claimed in claim 1, characterized in that the means in order to displace the mirror are constituted by a first membrane (2) supporting the mirror (1) which is kept spaced from the thorax by a support frame (21).

3. The device as claimed in claim 2, characterized in that it comprises a second membrane (20) fixed on the support frame in parallelism to the first membrane (2).

4. The device as claimed in claim 2 and claim 3, characterized in that the support frame (21) is made of a deformable material.

5. The device as claimed in any one of the preceding claims 1 through 4, characterized in that the first membrane (2) seals a cavity (12) and is pierced by a hole (22) in order to provide a communication between the space contained by the two membranes (2 and 20) and the cavity (12) and to create a high pass acoustic filter with the cavity (12) and the second membrane (20).

6. The device as claimed in any one of the preceding claims 1 through 5, characterized in that the mirror (1) has light from the light generator shone on it by means of a optical fiber (7).

7. The device as claimed in any one of the preceding claims 1 through 6, characterized in that the light rays reflected by the mirror (1) are transmitted to electrooptic means (5) with conversion by means of an optic fiber (8).

8. The device as claimed in any one of the preceding claims 1 through 7, characterized in that the sensor is kept in place by a girth lightly gripping the thorax.

9. The device as claimed in any one of the preceding claims 5 through 8, adapted for the synchronization of an apparatus for the acquisition of NMR images, characterized in that it comprises means in order to connect the optical device for the detection of the cardiac movements, said means for connection being connected with electronic treatment means (22,...., 34) furnishing, starting from the electric signal furnished by the electrooptic means (5) signals representing the cardiac, respiratory and 'budging' rhythm of the patient.

10. The device as claimed in claim 9, characterized in that the electronic treatment means comprise a first processing path (23,....29) in order to extract signals representing the respiratory rhythm and a second processing path (30,..., 34) in order to extract signals representing the cardiac rhythm.

11. The device as claimed in claim 10, characterized in that the first path comprises, connected in the following order and in series, an amplifier (23) with means for regulating its gain permitting the adaptation to the sensitivity of the sensor, a low pass filter (24) attenuating the cardiac noise in order to favor the signal coming from the thorax and due to respiration, a polarity inverter (25) in order to select the instant of synchronization to inspiration or expiration and a peak voltage detector (27) in order to obtain a precise and reproducible instant of the respiratory cycle.

12. The device as claimed in claim 10 and claim 11, characterized in that the second path comprises, connected in the following order and in series, an amplifier (30) with means for regulating its gain, a pass band filter (31) in order to suppress the respiratory signal and parasitic signals and/or ambient noise above the highest heart beat frequencies, an order of two analog deriving circuit (32) supplying pulses corresponding to the B1 and B2 noise signifying known electrocardiographic R and T waves and logic means for putting in form (34).

13. The device as claimed in claim 12, characterized in that it comprises logic means (34) for putting in form in order to supply an inhibition signal if the patient budges.

**Ansprüche**

1. Optische Vorrichtung zur gleichzeitigen Erfassung der Herz- und der Atmungsbewegungen, dadurch gekennzeichnet, daß sie einerseits einen Meßwertaufnehmer mit einem beweglichen Spiegel (1), der mit Mitteln (2, 3, 20, 21) zum Verschieben des Spiegels durch die Herz- und Thoraxbewegungen des Patienten gekoppelt ist, und andererseits einen Lichtgenerator, der mit einem Lichtleiter zum Beleuchten des Spiegels gekoppelt ist, elektrooptische Mittel (5), die ebenfalls mit diesem Lichtleiter gekoppelt sind, um die Intensität der vom Spiegel (1) reflektierten Lichtstrahlen in ein elektrisches Signal umzuwandeln, sowie Bearbeitungsmittel, um dieses elektrische Signal in die Herz- und Atmungsbewegungen darstellende Signale umzuwandeln, umfaßt.

2. Vorrichtung gemäß Anspruch 1, dadurch gekennzeichnet, daß die Mittel zum Verschieben des Spiegels durch eine den Spiegel (1) tragende erste Membran (2) gebildet werden, die durch einen Trägerrahmen (21) vom Thorax in einem Abstand gehalten wird.

3. Vorrichtung gemäß Anspruch 2, dadurch gekennzeichnet, daß sie eine zweite Membran (20) enthält, die parallel zur ersten Membran (2) am Trägerrahmen befestigt ist.

4. Vorrichtung gemäß den Ansprüchen 2 und 3, dadurch gekennzeichnet, daß der Trägerrahmen (21) aus einem verformbaren Material hergestellt ist.

5. Vorrichtung gemäß einem der Ansprüche 1 bis 4, dadurch gekennzeichnet, daß die erste Membran (2) einen Hohlraum (12) verschließt und ein Loch (22) aufweist, um einen Verbindung zwischen dem zwischen den zwei Membranen (2, 20) eingeschlossenen Raum und dem Hohlraum (12) herzustellen und um mit dem Hohlraum (12) und der zweiten Membran (20) ein akustisches Hochpaßfilter zu verwirklichen.

6. Vorrichtung gemäß einem der Ansprüche 1 bis 5, dadurch gekennzeichnet, daß der Spiegel (1) durch den Lichtgenerator mittels einer Lichtleitfaser (7) beleuchtet wird.

7. Vorrichtung gemäß einem der Ansprüche 1 bis 6, dadurch gekennzeichnet, daß die vom Spiegel (1) reflektierten Lichtstrahlen an die elektrooptischen Umwandlungsmittel (5) mittels einer Lichtleitfaser (8) übertragen werden.

8. Vorrichtung gemäß einem der Ansprüche 1 bis 7, dadurch gekennzeichnet, daß der Meßwertaufnehmer durch einen um den Thorax leicht gespannten Gurt gehalten wird.

9. Vorrichtung gemäß einem der Ansprüche 5 bis 8, die mit einem Kernspinresonanz-Bilderfassungsgerät synchronisiert ist, dadurch gekennzeichnet, daß sie Mittel zum Verbinden des Bilderfassungsgerätes mit der optischen Vorrichtung umfaßt, um die Herzbewegungen zu erfassen, wobei diese Mittel zum Verbinden mit elektronischen Bearbeitungsmitteln (22,...,34) verbunden sind, die anhand des von den elektrooptischen Mitteln (5) gelieferten elektrischen Signals Signale liefern, die die Herz- und Atmungsrhythmen und die "Bewegung" des Patienten darstellen.

10. Vorrichtung gemäß Anspruch 9, dadurch gekennzeichnet, daß die elektronischen Verarbeitungsmittel einen ersten Verarbeitungsweg (23,...,29) für die Aussonderung der den Atmungsrhythmus darstellenden Signale und einen zweiten Verarbeitungsweg (30,...,34) für die Aussonderung der den Herzrhythmus darstellenden Signale enthalten.

11. Vorrichtung gemäß Anspruch 10, dadurch gekennzeichnet, daß der erste Weg in dieser Reihenfolge in Reihenschaltung einen Verstärker (23) mit einstellbarer Verstärkung, die die Anpassung an die Empfindlichkeit des Meßwertaufnehmers gestattet, ein Tiefpaßfilter (24), das das Herzrauschen dämpft, um das im Thorax durch die Atmung entstehende Signal hervorzuheben, einen Polaritätsinvertierer (25) für die Wahl entweder der Einatmung oder der Ausatmung als Synchronisationszeitpunkt und einen Spitzenspannungsdetektor (27) für die Gewinnung eines genauen und wiedergebbaren Zeitpunkts des Atmungszyklus umfaßt.

12. Vorrichtung gemäß den Ansprüchen 10 und 11, dadurch gekennzeichnet, daß der zweite Weg in dieser Reihenfolge in Reihenschaltung einen Verstärker mit einstellbarer Verstärkung (30), ein Bandpaßfilter (31) für die Unterdrükkung des Atmungssignals und der parasitären Signale und/oder des Umgebungsrauschens oberhalb der höchsten Herzschlagfrequenzen, eine Analogdifferenzierschaltung (32) zweiter Ordnung, die zwei Impulse liefert, die dem Rauschen B1 und B2 entsprechen, das entsprechend die aus der Elektrokardiographie bekannten Wellen R und T kennzeichnet, und eine Formgebungslogik (34) umfaßt.

13. Vorrichtung gemäß Anspruch 12, dadurch gekennzeichnet, daß sie eine Formgebungslogik enthält, die im Fall der Bewegung des Patienten ein Sperrsignal liefert.

# FIG_1

# FIG_2

# FIG_3

# FIG_4

EP 0 271 369 B1

FIG_5

EP 0 271 369 B1

# FIG_6

80 ms

Sortie Q
Circuit 35

Sortie $\bar{Q}$
Circuit 36

0,4 s

Sortie
Porte 40

SIGNAL R

$\mathcal{T} = 40\,\mu s$

Sortie Q
Circuit 37

0,5 s

Sortie
Porte 43

Sortie $\bar{Q}$
Circuit 38

0,5 s

Sortie
Porte 46

SIGNAL T

# FIG_7

Circuit
35 { Signal normal

Signal bougé

Sortie Q̄
Circuit 36          0,4 s

Sortie
Porte 40                    Signal R

Sortie
Circuit 37          0,5 s

Sortie
Porte 43                    Signal T

Sortie
Porte 42

Sortie
Circuit 39          1 s
                    Signal
                    Inhibition